Europäisches Patentamt

European Patent Office

Office européen des brevets

(11) Publication number : **0 679 333 A2**

(12) **EUROPEAN PATENT APPLICATION**

(21) Application number : **95302469.2**

(22) Date of filing : **12.04.95**

(51) Int. Cl.⁶ : **A01N 25/28, A01N 43/80**

---

(30) Priority : **28.04.94 US 234803**

(43) Date of publication of application :
**02.11.95 Bulletin 95/44**

(84) Designated Contracting States :
**AT BE CH DE DK ES FR GB GR IE IT LI LU NL PT SE**

(71) Applicant : **ROHM AND HAAS COMPANY**
**100 Independence Mall West**
**Philadelphia, Pennsylvania 19106-2399 (US)**

(72) Inventor : **El A'mma, Beverly Jean**
**133 Bavington Road**
**Perkiomenville, Pennsylvania 18074 (US)**
Inventor : **Redlich, George Harvey**
**3046 Taft Road**
**Norristown, Pennsylvania 19403 (US)**

(74) Representative : **Smith, Julian Philip Howard et al**
**Rohm and Haas (UK) Limited,**
**European Operations Patent Dept.,**
**Lennig House,**
**2 Masons Avenue**
**Croydon CR9 3NB (GB)**

---

(54) **Non-sensitizing biocide composition.**

㉗ The skin sensitization potential of 3-isothiazolone biocides in loci such as water-based marine antifouling paints or decorative paint is reduced by encapsulating the biocide in polyurea particles.

This invention relates to compositions containing a biocide, usually to control mildew or prevent fouling.

Compositions such as paints, varnishes, mastics, sealants, adhesives, caulks, metal working fluids and the like, typically contain a biocide to control mildew or prevent fouling. The 3-isothiazolone class of biocides has been most effective in these applications. For example, in coatings and other compositions which are subject to mildew, 2-n-octyl-3-isothiazolone is a common mildewcide.

In the field of marine paint, 4,5-dichloro-2-n-octyl-3-isothiazolone has proven to be an extraordinarily effective biocidal active ingredient to prevent fouling of the dried film on a marine substrate such as ships, piers, fishnets, rigs, and the like. One problem with this isothiazolone is that painters must be careful not to be exposed to wet paint containing it because it causes skin sensitization. Much research has been devoted to this problem because it has limited commercial acceptance of the isothiazolone, not only in marine paints, but also in decorative coatings. Nothing in the prior art suggests a method for solving the sensitization problem with 3-isothiazolone compounds used as biocides.

Prior attempts to encapsulate isothiazolone biocides involved polymerizing methyl methacrylate and methacrylic acid in the presence of the active ingredient and then incorporating the encapsulated biocide in paint to improve its heat aged stability. Redlich et al, U.S. 5,225,279 taught such encapsulation for the purpose of controlling or reducing the release rate of the active ingredient.

It is also known to encapsulate agricultural chemicals to control release rate with polyurea encapsulant. However, no one has heretofore solved the important problem of sensitization due to exposure to 3-isothiazolone active ingredient in compositions.

It is therefore an object of the invention to provide a method of reducing the sensitization potential of 3-isothiazolone biocides when used in compositions. Accordingly the present invention provides in one aspect a composition comprising particles of polyurea encapsulating 3-isothiazolone. The invention also provides in another aspect a method of reducing the skin sensitizing effect of a composition containing a 3-isothiazolone, comprising encapsulating said 3-isothiazolone in polyurea particles and dispersing said particles in the composition. A further aspect of the invention is the use of such particles to reduce the skin sensitization potential of 3-isothiazolones.

The polyurea encapsulant (or shell) is generally a reaction product of one or more polyisocyanates and one or more polyfunctional amines. Preferred polyisocyanates are polymethylene polyphenylisocyanate (PAPI); 2,6-toluene diisocyanate (TDI); bis(4-isocyanatocyclohexyl)methane; hexamethylene diisocyanate (HDI); para-phenylene diisocyanate; meta-phenylene diisocyanate; naphthalene-1,5-diisocyanate; diphenylmethane-4,4-diisocyanate (MDI); triphenylmethane-4,4',4''-triisocyanate; isopropyl benzene diisocyanate; and isophorone diisocyanate (IPDI). Preferred polyfunctional amines are ethylene diamine; diethylene triamine; triethylene tetramine; tetraethylene pentamine; pentaethylene hexamine; phenylenediamine; toluene diamine; 1,3,5-benzene triamine•3HCl; 2,4,6-triamino toluene•3HCl; polyethylene amine; 1,3,6-triaminonaphthalene; 3,4,5,8-tetraamino-anthroquinone; hexamethylene diamine; and piperazine.

The polyisocyanate and the polyamine are polymerized in the presence of the 3-isothiazolone active ingredient, preferably by interfacial condensation wherein the isocyanate and active ingredient are in the oil phase and the polyfunctional amine is in the aqueous phase. It is preferred to add the organic phase to the aqueous phase in a mixer and then transfer the resultant dispersion to a reaction container while maintaining the temperature at about 30-40°C until the reaction is complete and solid particles are formed which are separated and allowed to air dry. Typical particles contain between 25 and 90% active ingredient by weight and have a particle size of about 1-100 microns.

While any of the 3-isothiazolones are suitable, the preferred 3-isothiazolone compounds are 2-n-octyl-3-isothiazolone and 4,5-dichloro-2-n-octyl-3-isothiazolone. The latter is preferred for marine applications and both are preferred for mildewcide applications, usually in decorative or architectural coatings.

The compositions in which the particles are used may be any in which 3-isothiazolones are useful as biocides, including caulk; sealant; paint; varnish; mastic; adhesive; film forming polymers; wood impregnant; plastic; and rubber.

Suitable film forming polymers for the architectural, decorative or marine coating compositions are any of the ones which are used in water based paints, preferably of the acrylic or vinyl acetate latex copolymer type.

In the case of coatings, the encapsulated particles can be incorporated by mixing with the film forming polymer, water and optionally a pigment.

Isothiazolones can be encapsulated in a variety of polymer matrices by standard procedures. When compared to other encapsulating materials, only polyurea encapsulation of the isothiazolone provided reduced skin sensitization. This was unexpected.

In the following examples all parts and percentages are by weight unless otherwise indicated.

2

EXAMPLE 1 - Isothiazolone Encapsulation A

The isothiazolone was encapsulated by interfacial polymerization.

Three solutions were prepared, an aqueous phase, an organic phase, and a cross-linking solution. The aqueous phase was prepared by combining 991.6 g de-ionized water; 0.20 g sodium diethyl hexyl sulfosuccinate, available commercially as Monowet MO-70E®; and 8.20 g polyvinyl alcohol, 88% hydrolyzed from polyvinyl acetate, available commercially as Airvol 205®. The organic phase was prepared by combining 141.25 g 4,5-dichloro-2-n-octyl-3-isothiazolone; 40.0 g xylene; 12.0 g dioctylphthalate; and 148.8 g 4,4'-dipenylmethane diisocyanate, available commercially as Mondur MRS®. The cross-linking solution was prepared by combining 17.04 g ethylene diamine; 23.64 g triethylene tetramine; and 400.00 g de-ionized water.

The organic phase was added to the aqueous phase and the mixture homogenized for 30 seconds at 15000 rpm with a Tekmar Tissumizer®. The resultant dispersion was transferred to a 2 L. round bottom flask fitted with an overhead stirrer, a thermometer, and a temperature regulator. The dispersion was stirred at a rate of 400 rpm. The cross-linking solution was added to the stirred dispersion with a resulting rise in temperature to 35°C. The reaction was allowed to stir for 4 hours at 35°C.

At the end of 4 hours, the pH of the reaction mixture was found to be 7.8. The resultant solid particles were removed from the solution by vacuum filtration and allowed to air dry. Analysis of the particles showed 44.7% 4,5-dichloro-2-n-octyl-3-isothiazolone. The mean particle size was found to be 18 microns.

EXAMPLE 2 - Isothiazolone Encapsulation B

The isothiazolone was encapsulated by interfacial polymerization.

Three solutions were prepared, an aqueous phase, an organic phase, and a cross-linking solution. The aqueous phase was prepared by combining 991.6 g de-ionized water; 0.20 g sodium diethyl hexyl sulfosuccinate, available commercially as Monowet MO-70E®; and 8.20 g polyvinyl alcohol, 88% hydrolyzed from polyvinyl acetate, available commercially as Airvol 205®. The organic phase was prepared by combining 141.25 g 4,5-dichloro-2-n-octyl-3-isothiazolone; 40.0 g xylene; 12.0g diisodecylphthalate; and 48.8g 4,4'-diphenylmethane diisocyanate, available commercially as Mondur MRS®. The cross-linking solution was prepared by combining 17.04g ethylene diamine; 23.64 g triethylene tetramine; and 400.00g de-ionized water.

The organic phase was added to the aqueous phase and the mixture homogenized for 3 minutes at 6000 rpm with a Ross Micro-mixer Emulsifier®. The resultant dispersion was transferred to a 3 L. round bottom flask fitted with an overhead stirrer, a thermometer, and a temperature regulator. The dispersion was held at 40°C for one hour at a stir speed of 400 rpm prior to the addition of the cross-linking solution. The cross-linking solution was added to the stirred dispersion at 40°C. The reaction was allowed to stir for 4 hours at 40°C.

At the end of 4 hours, the pH of the reaction mixture was found to be 9.2. The resultant solid particles were removed from the solution by vacuum filtration and allowed to air dry. Analysis of the particles showed 48% 4,5-dichloro-2-n-octyl-3-isothiazolone. The mean particle size was found to be 8 microns.

EXAMPLE 3 - Comparative Encapsulating Materials and Methods

The relative release rates ("RRR") of a polyurea-encapsulated isothiazolone of the invention and other polymer encapsulated isothiazolones were determined.

A dispersion of encapsulated 4,5-dichloro-2-n-octyl-3-isothiazolone was prepared by adding sufficient encapsulated isothiazolone to provide a total of 26 mg of 4,5-dichloro-2-n-octyl-3-isothiazolone to 360 g of an aqueous soap solution (0.2% sodium diethyl hexyl sulfosuccinate). The actual amount of each encapsulated isothiazolone added depended upon the loading of the isothiazolone in each polymer matrix.

A hollow fiber dialysis membrane set containing 176 fibriles, in the shape of a "U", was placed into the dispersion. Aqueous soap solution was pumped from a reservoir, containing 1200 g of aqueous soap solution, through the hollow fibers, at a rate of 2.9 - 3.1 ml/min, back into the reservoir. The isothiazolone diffused from the encapsulation matrix into the aqueous phase of the dispersion, and then through the hollow fiber membrane into the reservoir system. The accumulation of isothiazolone in the reservoir system was monitored by taking aliquots of the reservoir at various times. The aliquots were analyzed for 4,5-dichloro-2-n-octyl-3-isothiazolone by HPLC.

In the case of unencapsulated 4,5-dichloro-2-n-octyl-3-isothiazolone, 26 mg of the isothiazolone were added to the aqueous soap solution to form the dispersion and the experiment performed as described above. The RRR for unencapsulated 4,5-dichloro-2-n-octyl-3-isothiazolone was defined to be 1.0. The RRR's for the various forms of encapsulated 4,5-dichloro-2-n-octyl-3-isothiazolone were determined relative to this.

The polyurea encapsulated 4,5-dichloro-2-n-octyl-3-isothiazolone of the invention was prepared as in Example 1. The comparative forms of encapsulated 4,5-dichloro-2-n-octyl-3-isothiazolone were prepared by known methods: styrene/divinylbenzene ("STY/DVB") expanded with DVB, by an expanded seed process vinyl polymerization; polyamide ("pAmide") by interfacial polymerization; polyvinyl alcohol ("pVinyl Alcohol") by spray drying; polyvinylchloride ("PVC")/dispersion by dissolving isothiazolone into methylenedichloride/PVC solution dispersed into water followed by solvent evaporation; trisacetoacetate/dispersion by interfacial polymerization; and cross-linked acrylic dispersion by vinyl polymerization.

The results are reported in Table 1.

Table 1 - Relative Release Rates

From Particles of Invention and From Comparative Articles

| Test Compound | % Isothiazolone Encapsulated | RRR |
|---|---|---|
| Control | | |
| 4,5-dichloro-2-n-octyl-3-isothiazolone | --- | 1.0 |
| Invention | | |
| pUrea (interfacial polymerization) | 44.7 | 0.09 |
| Comparative | | |
| Sty/DVB, expanded with DVB (vinyl polymerization) | 37.5 | 0.31 |
| pAmide (interfacial polymerization) | 73.8 | 0.37 |
| pVinyl Alcohol (spray drying) | 65.7 | 0.31 |
| PVC (Dispersion of AI into PVC) | 19.8 | 0.36 |
| Trisacetoacetate Dispersion (interfacial polymerization | 25.0 | 0.61 |
| X-linked Acrylic Dispersion (vinyl polymerization) | 31.7 | 0.60 |

From the above data, it can be seen that polyurea encapsulation of the isothiazolone greatly reduces the release rate of the isothiazolone and is much more effective than other encapsulating compositions.

EXAMPLE 4 - Efficacy Determination of Encapsulated Isothiazolone

A series of paints of the formulation set forth in Table 2 were prepared and tested for efficacy (exterior exposure results) and lymph node assay (sensitization) from the isothiazolone biocide. The isothiazolone biocide was introduced as either particles prepared in accordance with Example 1 or in accordance with comparative encapsulation methods in an amount sufficient to introduce 2000 ppm or 1000 ppm of active ingredient based on total paint formulation.

An exterior exposure study to demonstrate encapsulation, and that reduced release rate does not effect long term efficacy was performed. Panels were painted with the paint described in Table 2, to which was added the proper amount of active ingredient. After air drying, the panels were placed on exposure at a farm in Glen St. Mary, Florida, in a North-vertical position.

4

Table 2

| - Paint Formulation | | | |
|---|---|---|---|
| Materials | Function | Weight Ratio | Parts Per Hundred (Volume Basis) |
| Natrosol 250 MHR (HEC) | Thickener | 3.0 | 0.26 |
| Ethylene Glycol | Anti-freeze | 25.0 | 2.65 |
| Water | Diluent | 120.0 | 14.40 |
| Tamol 960 | Dispersant | 7.1 | 0.67 |
| Potassium Tripolyphosphate | Codispersant | 1.5 | 0.07 |
| Triton CF-10 | Surfactant | 2.5 | 0.28 |
| Colloid 643 | Defoamer | 1.0 | 0.13 |
| Propylene glycol | Wet edge aid | 34.0 | 3.94 |
| Ti-Pure R-902 ($TiO_2$) | Opacifier | 225.0 | 6.57 |
| Minex 4 | Extender | 159.4 | 7.32 |
| Icecap K | Extender | 50.0 | 2.33 |
| Attagel 50 | Anti-setting | 5.0 | 0.25 |

The above ingredients were milled for about 15 minutes in a Cowles Dissolver at 3800-4500 rpm and let down at slower speed as follows:

| Rhoplex AC-64 (60.5%) | Latex Binder | 305.9 | 34.10 |
|---|---|---|---|
| Colloid 643 | Defoamer | 3.0 | 0.39 |
| Texanol | Coalescent | 9.3 | 1.17 |
| Mildewcide | | 2 to 7 | 0.25 - 1.00 |
| Ammonia, 28% | pH Control | 2.0 | 0.27 |
| Water | Viscosity | Enough for 90 to 95 KU and | |
| 2.5% Natrosol 250 MHR | Adjustment | to adjust to 100 gal total | |
| | Totals: | ca. 1162 | 100.00 |

| Formulation Constants | |
|---|---|
| Pigment Volume Content, % | 45.4 |
| Volume Solids, % | 36.2 |
| Initial viscosity, KU | 90 to 95 |
| pH | 9.5 |

The overall mildew ratings after 2 and 6 months are shown in Table 3. The ratings are from 0 (completely covered with mildew) to 10 (no mildew growth).

Table 3

| - Exposure Results, Decorative Paint | | | |
|---|---|---|---|
| | Overall Mildew Rating (2 month / 6 month) | | |
| Sample | 0 ppm | 1000 ppm | 2000 ppm |
| Blank Control | 5.6 / 0.0 | | |
| Unencapsulated isothiazolone | | 6.0 / 6.3 | 9.7 / 9.3 |
| pUrea (invention) | | 5.6 / 5.6 | 9.6 / 9.1 |
| pAmide (comparative) | | 9.0 / 7.3 | 9.7 / 9.6 |
| Sty/DVB (comparative) | | 9.0 / 6.7 | 8.7 / 9.3 |

Thus, the polyurea encapsulated isothiazolone is shown to be as efficacious as the unencapsulated isothiazolone.

EXAMPLE 5- Evaluation of Sensitization Potental

The mouse lymph node assay is used as a rapid screening technique for the determination of skin sensitizers. Direct epicutaneous application to the ears is an appropriate route of administration for assessing the contact allergenic potential of a test substance. $^3$H-Thymidine incorporation into the DNA of lymphocytes, isolated from the auricular lymph nodes, results from proliferation of the cells after application of a contact sensitizer to the dorsal and ventral sides of the ears. Measurement of $^3$H-thymidine uptake by the cells is an objective and quantifiable response. Therefore, the more $^3$H-thymidine incorporated, the greater the skin sensitization potential for the compound tested.

The paint formulations of Example 4 were evaluated in a lymph node assay according to the method of Gerberick, et al., Fundam. Appl. Toxicol. 19, 438-445 (1992). Varying concentrations of the paint formulations of Example 4 were applied to the ears (dorsal and ventral sides or dorsal side only) of 4 to 5 mice between one and four days. On day five, $^3$H-thymidine (20 mCi/mouse) was administered intravenously to all mice. Five hours post-injection, the animals were euthanized via carbon dioxide asphyxiation and the auricular lymph nodes were removed. The lymph nodes were homogenized in 5% trichloroacetic acid and cell proliferation in the draining auricular lymph nodes was determined by $^3$H-thymidine incorporation into the DNA fraction. This was determined by liquid scintillation counting.

The results of the sensitization testing are reported in Table 4 wherein all samples contained 4000 ppm of 4,5-dichloro-2-octyl isothiazolone and disintegrations per minute (DPM) are reported. Lower DPM represents less sensitization (less $^3$H-thymidine incorporated). Trial 2 was confirmatory.

Table 4

| Encapsulation Method | DPM Trial 1 | DPM Trial 2 |
|---|---|---|
| Controls | | |
| No Biocide | 12,872 | 10,765 |
| Unencapsulated isothiazolone | 24,052 | 24,922 |
| Invention | | |
| Example 1 | 13,774 | 11,013 |
| Comparative | | |
| pAmide | 25,616 | |
| st/DVB | 22,543 | |

From the above data, it was surprisingly found that the polyurea encapsulated isothiazolone of the invention had a much lower skin sensitization potential than other encapsulents or than the unencapsulated isothiazolone.

**Claims**

1. Composition comprising particles of polyurea encapsulating 3-isothiazolone.

2. Composition according to claim 1 wherein said 3-isothiazolone comprises 4,5-dichloro-2-n-octyl-3-isothiazolone or 2-n-octyl-3-isothiazolone.

3. Composition according to claim 1 or 2 wherein said polyurea is formed by polymerization of one or more polyisocyanates and one or more polyamines in the presence of said 3-isothiazolone.

4. Composition according to claim 3 wherein said polyamine comprises diethylene, triethylene or tetraethylene amines.

5. Composition according to claim 3 or 4 wherein said polyisocyanate comprises polymethylene polyphenylisocyanate (PAPI); 2,6-toluene diisocyanate (TDI); bis(4-isocyanatocyclohexyl)methane; hexamethylene diisocyanate (HDI); para-phenylene diisocyanate; meta-phenylene diisocyanate; naphthalene-1,5-diisocyanate; diphenylmethane-4,4-diisocyanate (MDI); triphenylmethane-4,4',4"-triisocyanate; isopropyl benzene diisocyanate; or isophorone diisocyanate (IPDI).

6. Composition according to any preceding claim wherein said particles have a diameter of less than 100 microns, preferably less than 40 microns.

7. Composition according to any preceding claim wherein said particles comprise from 25 to 90% by weight 3-isothiazolone.

8. A caulk; sealant; paint; varnish; mastic; adhesive; film-forming polymer; wood impregnant; plastic; metalworking fluid; cosmetic composition; or rubber, containing a composition as defined in any preceding claim.

9. A marine anti-fouling paint comprising a film forming polymer, a pigment, and a composition as defined in any of claims 1 to 7, wherein the isothiazolone is 4,5-dichloro-2-n-octyl-3-isothiazolone.

10. A decorative paint comprising a film-forming polymer, a pigment, and a composition as defined in any of claims 1 to 7, wherein the particles of said composition have a diameter of less than 20 microns and comprise 0.03 to 0.5% by weight of the paint.

11. A substrate, preferably a marine structure such as a ship, floating platform, pier, rig or fishnet, coated with a paint as defined in claim 9 or 10.

12. Method of reducing the skin sensitizing effect of a composition containing a 3-isothiazolone, comprising encapsulating said 3-isothiazolone in polyurea particles and dispersing said particles in the composition.

13. Use of a composition as defined in any of claims 1 to 7 to reduce the skin sensitizing effect of 3-isothiazolones.